# FASCICULE DE BREVET EUROPEEN

(11) **EP 1 563 810 B1**
(45) Date de publication et mention de la délivrance du brevet: **31.03.2010**
(21) Numéro de dépôt: 05356029.8
(22) Date de dépôt: 09.02.2005
(51) Int. Cl.: A61F 2/46, A61B 19/00

(54) **Dispositif chirurgical d'implantation d'une prothèse totale de hanche**
Chirurgische Vorrichtung zum Einsetzen einer Totalhüftprothese
Surgical apparatus for implanting a total hip prosthesis

(30) Priorité: 10.02.2004 FR 0401281
(43) Date de publication de la demande: 17.08.2005
(73) Titulaire: TORNIER, 38330 Saint-Ismier (FR)
(72) Inventeur: Laffargue, Philippe, 59110 La Madeleine (FR); Migaud, Henri, 59000 Lille (FR); Puget, Jean, 31500 Toulouse (FR); Giraud, François, 59175 Templemars (FR); Tabutin, Jacques, 06110 Le Cannet (FR)
(74) Mandataire: Myon, Gérard Jean-Pierre

(56) Documents cités:
- WO-A-02/02028
- WO-A-02/080824
- WO-A-2004/001569
- DE-U- 20 016 635
- DE-U- 20 213 711
- DE-U- 20 304 153
- DE-U- 20 315 005
- FR-A- 2 770 128
- US-A1- 2002 077 540
- US-A1- 2003 153 829
- US-B1- 6 205 411

## Description

La présente invention concerne un dispositif chirurgical d'implantation d'une prothèse totale de hanche.

Une prothèse totale de hanche comporte de façon classique, d'une part, une partie fémorale constituée d'une tige à une extrémité de laquelle est fixée une tête fémorale délimitant une surface articulaire convexe globalement sphérique et, d'autre part, une partie cotyloïdienne à fixer à l'os du bassin, comportant par exemple une cupule métallique cotyloïdienne en forme de demi-sphère, à l'intérieur de laquelle est logé un insert en matière plastique ou en céramique dans lequel vient s'articuler la tête fémorale.

Lors de la pose d'une telle prothèse totale de hanche, le chirurgien implante d'une part la tige fémorale à l'intérieur d'une cavité creusée longitudinalement dans l'os du fémur et, d'autre part, la partie cotyloïdienne de la prothèse dans une cavité globalement hémisphérique creusée dans l'os du bassin. La direction d'implantation de la tige fémorale dans l'os du fémur est globalement imposée par la forme allongée de l'os du fémur alors que le chirurgien dispose d'une plus grande liberté pour choisir la position d'implantation de la partie cotyloïdienne dans la cavité creusée dans l'os du bassin. Le choix de cette position influe directement sur la position de l'axe de révolution du cône de mobilité théorique de la prothèse implantée, cet axe de révolution correspondant en fait à l'axe de révolution de la cupule hémisphérique solidarisée à l'os du bassin.

On a remarqué que le positionnement du cotyle au niveau de la zone d'implantation du bassin a des conséquences sur le comportement mécanique de la prothèse implantée. Plus précisément, lorsque la prothèse est articulée selon des mouvements d'amplitude extrême, notamment selon des mouvements combinant des déplacements élémentaires de la hanche en flexion-extension, en abduction-adduction et/ou en rotation interne-rotation externe, il arrive qu'elle soit sollicitée en dehors de son cône de mobilité prothétique, provoquant alors un contact d'appui entre le col fémoral de la prothèse et le bord du cotyle. Dans ces conditions, il arrive que la prothèse se luxe.

US-6,205,411 propose une méthode de pose d'une prothèse de hanche, qui aide le chirurgien à implanter la prothèse en vue de limiter les risques de luxations ultérieures de la prothèse en s'adaptant à l'anatomie du patient traité. A cet effet, il est prévu d'utiliser d'une part, un simulateur pré-opératoire de la cinématique biomécanique de la hanche du patient munie de façon virtuelle de la prothèse à implanter ultérieurement et, d'autre part, un dispositif de guidage peropératoire des gestes du chirurgien pour poser la prothèse, ce dispositif étant commandé à partir des résultats issus de la simulation biomécanique menée au moyen du simulateur. Pour permettre au simulateur de déterminer un positionnement adéquat de la prothèse à implanter, il est nécessaire de lui fournir une cartographie complète et détaillée de la structure osseuse du patient, notamment au moyen de techniques tomographiques non-invasives. Toutes ces données sont traitées informatiquement pour re-créer, de manière virtuelle, l'anatomie osseuse du patient et pour ensuite, toujours en pré-opératoire, simuler son comportement avec une prothèse virtuelle. Cette méthode nécessite donc de très importants moyens informatiques qui sont coûteux, ainsi qu'un grand nombre de données pré-opératoires, ce qui allonge la durée et le coût d'hospitalisation du patient.

Le but de la présente invention est de proposer un dispositif chirurgical qui assiste de manière plus simple, plus rapide et plus économique le chirurgien lors de la chirurgie d'implantation d'une prothèse totale de hanche en vue de limiter les risques de luxations ultérieures de la prothèse en s'adaptant au mieux à l'anatomie de chaque patient traité.

A cet effet, l'invention a pour objet un dispositif chirurgical d'implantation d'une prothèse totale de hanche en accord avec la revendication 1.

En utilisant le dispositif selon l'invention, le chirurgien peut, au cours de la chirurgie proprement dite, comparer le cône de mobilité prothétique associé à la prothèse à implanter avec les différentes positions mesurées de la direction prothétique fémorale considérée, ces positions correspondant de préférence à des configurations articulaires extrêmes de la hanche du patient opéré, à savoir des configurations articulaires combinant des mouvements de flexion/extension, d'abduction/adduction et/ou de rotation interne/externe, telles que par exemple la configuration en tailleur qui combine des mouvements de flexion, d'abduction et de rotation externe. Le chirurgien choisit alors, durant l'intervention chirurgicale de pose de la prothèse, une direction préférentielle pour implanter le cotyle de la prothèse, permettant la sollicitation ultérieure de la prothèse jusque dans ces configurations articulaires extrêmes sans risquer sa luxation. Autrement dit, le dispositif chirurgical selon l'invention permet au chirurgien de déterminer cette direction d'implantation préférentielle, ou plusieurs de ces directions préférentielles, que le chirurgien va ensuite respecter à la fin de l'intervention chirurgicale pour implanter le cotyle de la prothèse.

D'autres caractéristiques de ce dispositif, prises isolément ou selon toutes les combinaisons techniquement possibles, sont énoncées aux revendications dépendantes 2 à 10.

Avec le dispositiv selon l'invention on peut effectuer une méthode chirurgicale d'implantation d'une prothèse totale de hanche, dans laquelle, en peropératoire, successivement :
- on repère dans l'espace l'os du fémur et l'os du bassin d'un patient à traiter,
- on mesure et on mémorise plusieurs positions d'une direction prothétique fémorale donnée par rapport à une cavité du bassin,
- on compare ces positions mesurées avec le cône de mobilité de la prothèse à implanter, la position de l'axe de révolution de ce cône étant ajustée par rapport à la cavité au cours de l'intervention chirurgicale,
- on détermine au moins une position préférentielle de cet axe de révolution, et
- on impacte une partie cotyloïdienne de la prothèse dans la cavité du bassin suivant ladite position préférentielle.

L'invention sera mieux comprise à la lecture de la description qui va suivre, donnée uniquement à titre d'exemple et faite en se référant aux dessins sur lesquels :
- la figure 1 est_ une vue schématique d'une partie d'un dispositif chirurgical selon l'invention, appliqué à la hanche d'un patient à opérer ;
- la figure 2 est une vue éclatée en perspective d'une prothèse totale de hanche à implanter au moyen du dispositif de la figure 1 ;
- la figure 3 est une vue schématique en élévation d'une autre partie du dispositif selon l'invention, en cours d'utilisation sur le fémur de la hanche du patient ;
- la figure 4 est une vue en coupe d'une cavité cotyloïdienne creusée dans le bassin de la hanche du patient, au moyen du dispositif selon l'invention ;
- la figure 5 est une vue schématique illustrant la mise en place, à l'intérieur de la cavité de la figure 4, d'un composant fémoral fantôme du dispositif selon l'invention ;
- la figure 6 représente une vue schématique en perspective affichée à l'intention du chirurgien par le dispositif selon l'invention ;
- la figure 7 est une vue en perspective d'un composant cotyloïdien fantôme du dispositif selon l'invention ; et
- la figure 8 représente une autre vue schématique en perspective affichée à l'intention du chirurgien par le dispositif selon l'invention.

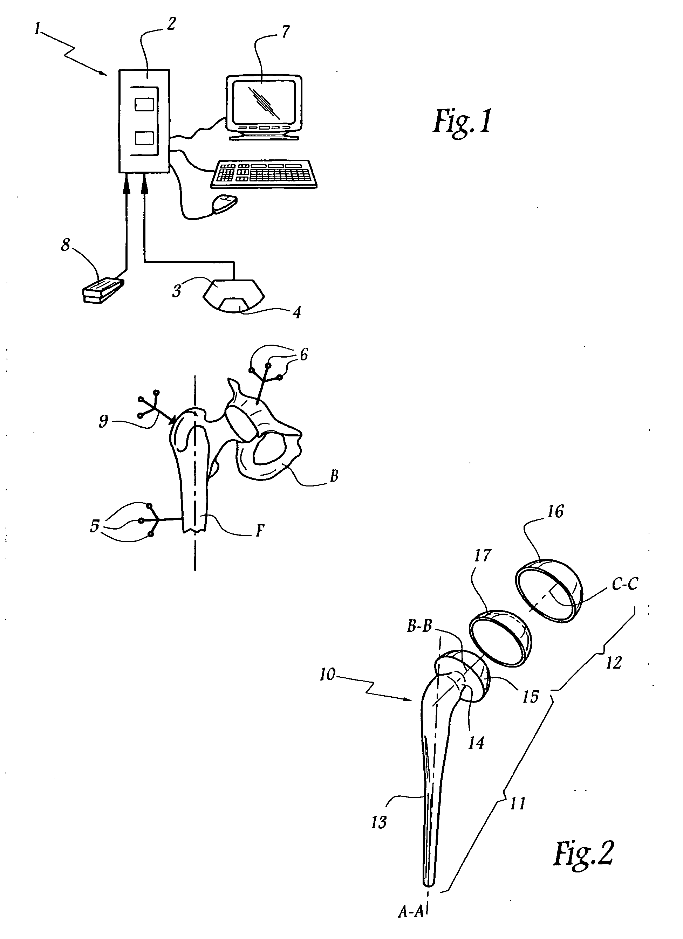

Le dispositif chirurgical 1 de la figure 1 comprend un ordinateur 2 associé à une unité d'émission et de réception de rayonnements infra-rouge. Cette unité comporte un capteur 3 relié à l'ordinateur et une source d'émission infra-rouge 4 couvrant le champ opératoire dans lequel est représentée en partie une hanche d'un patient à traiter. La hanche comprend la partie supérieure d'un fémur F et une partie correspondante de l'os du bassin B.

Pour permettre à l'ordinateur 2 de repérer dans l'espace les os du fémur F et du bassin B, le dispositif 1 comporte des groupes respectifs de marqueurs 5 et 6 qui renvoient, de façon passive, le rayonnement infra-rouge en direction du capteur 3. Chaque groupe de marqueurs 5 ou 6 forme un système de marquage tri-dimensionnel permettant à l'ensemble ordinateur 2 - capteur 3 de suivre dans l'espace les déplacements respectifs du fémur et du bassin. L'utilisation de tels marqueurs étant bien connue dans le domaine de l'orthopédie, ils ne seront donc pas décrits ici plus avant.

Chaque groupe de marqueurs 5 ou 6 est fixé à l'os du fémur ou du bassin au moyen d'une ou de plusieurs broches rigides. Comme on le comprendra après, ces broches sont placées de manière à laisser les marqueurs visibles pour le capteur 3 aussi bien lorsque l'articulation de la hanche est réduite (comme sur la figure 1) que lorsque elle est luxée.

L'ordinateur 2 du dispositif 1 est également associé à un ou plusieurs écrans 7 à même d'afficher des informations utiles au chirurgien, notamment les informations relatives à la position des os F et B et d'autres données décrites plus loin, de préférence sous forme de représentations graphiques tri-dimensionnelles comme détaillé ci-après.

Le dispositif 1 comporte également des moyens de commande 8 par exemple sous forme d'une pédale à même d'être actionnée par le pied du chirurgien.

Le dispositif chirurgical 1 comporte en outre d'autres composants qui vont être détaillés ci-après lors de la description d'un exemple détaillé d'utilisation du dispositif en vue de l'implantation d'une prothèse totale de hanche 10 représentée seule sur la figure 2. Cette prothèse est constituée d'une partie fémorale 11 à implanter dans l'os du fémur F et d'une partie cotyloïdienne 12 à implanter dans l'os du bassin B. Plus précisément, la partie fémorale 11 comporte une tige 13 d'axe longitudinal A-A, destinée à être logée et retenue dans une cavité diaphysaire creusée dans le canal médullaire du fémur F. L'extrémité supérieure de cette tige se prolonge, suivant une direction inclinée par rapport à l'axe A-A, sous la forme d'un col 14 à l'extrémité libre duquel est fixée une tête sphérique tronquée 15, d'axe de symétrie B-B et correspondant globalement à l'axe longitudinal du col 14.

La partie cotyloïdienne 12 comporte un cotyle 16 sous forme d'une cupule métallique sensiblement hémisphérique destinée à être solidarisée à l'os du bassin B. L'axe de révolution de la surface interne concave du cotyle est noté C-C. A l'intérieur de cette cupule est prévu d'être logé de manière fixe un insert 17 également hémisphérique d'axe de révolution C-C, constitué d'une matière plastique ou céramique. La surface intérieure de l'insert 17 est conformée de manière sensiblement complémentaire à la surface extérieure de la tête fémorale 15, de manière à ce que cette dernière s'articule à la manière d'une rotule par rapport à l'ensemble cotyloïdien 12.

La prothèse 10 décrite ci-dessus n'est donnée qu'à titre d'exemple et d'autres prothèses, de géométries et/ou de natures différentes, peuvent être implantées au moyen du dispositif 1, suivant la méthode chirurgicale d'implantation décrite ci-après. En particulier, l'invention s'applique à la pose de prothèses dont la partie cotyloïdienne est constituée d'une seule cupule à cimenter à l'os du bassin et dans laquelle est articulée directement la tête fémorale prothétique ou dont la partie cotyloïdienne comporte, en plus d'une première cupule métallique à fixer au bassin, une seconde cupule montée dans cette première cupule de façon articulée (on parle alors d'un ensemble cotyloïdien à double mobilité). Dans tous les cas, la partie cotyloïdienne de la prothèse délimite un axe de révolution pour la surface interne concave de la cupule à fixer au bassin, analogue à l'axe C-C.

Dans un premier temps, le chirurgien incise le patient et recueille un certain nombre de données relatives à la géométrie anatomique des os du fémur F et du bassin B. A cet effet, différents moyens d'acquisition de ces données sont envisageables. A titre d'exemple, le chirurgien utilise un palpeur 9 repéré par l'ensemble ordinateur 2 - capteur 3 et préalablement étalonné. Ce palpeur est amené sur des lieux remarquables des os et, à chacune de ces mises en place, le chirurgien actionne la pédale de commande 8 de manière à ce que l'ordinateur 2 enregistre la position du palpeur et par là en déduise les caractéristiques anatomiques du fémur F et du bassin B. A partir de ces données et du suivi des marqueurs 5 et 6, l'ordinateur 2 est capable de repérer dans l'espace les os du fémur et du bassin.

Durant cette étape d'acquisition de données, l'articulation de la hanche est successivement luxée et réduite, les marqueurs réfléchissants 5 et 6 restant visibles pour le capteur 3.

Dans un deuxième temps, la tête anatomique du fémur F est, si nécessaire, réséquée.

Dans un troisième temps, une cavité, destinée à recevoir ultérieurement la tige fémorale 13 de la prothèse 10, est creusée dans la diaphyse du fémur F. A cet effet, le chirurgien utilise d'abord une broche rigide non représentée qu'il introduit dans le canal médullaire anatomique du fémur et qu'il repère dans l'espace au moyen de l'ensemble ordinateur 2 - capteur 3 en venant par exemple palper une extrémité de cette broche portant un relief prédéterminé. Le chirurgien positionne alors la broche ainsi repérée de manière à ce qu'elle s'étende suivant une direction diaphysaire X-X destinée à constituer l'axe d'implantation de la partie fémorale 11 de la prothèse. Cette direction diaphysaire X-X est par exemple arbitrairement choisie par le chirurgien, en fonction de la forme et de l'état du fémur. Lorsque cette broche est positionnée convenablement, le chirurgien actionne la pédale de commande 8 et l'ordinateur 2 mémorise la position de l'axe X-X, notamment par rapport au fémur F.

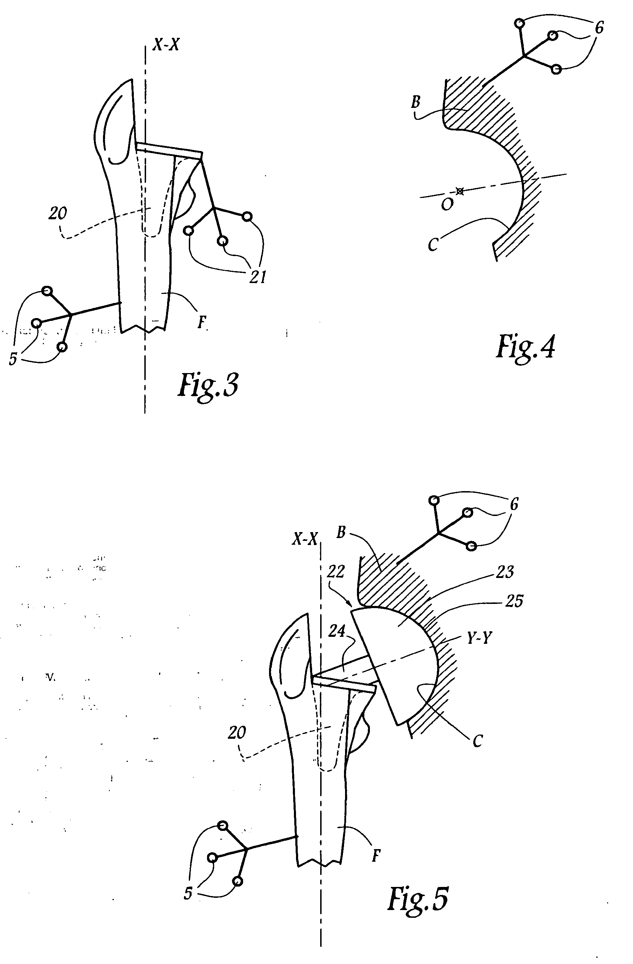

Après avoir retiré la broche, le chirurgien utilise ensuite une râpe fémorale 20 représentée en pointillés sur la figure 3. Cette râpe 20 présente une surface active de forme sensiblement identique à la tige fémorale 13. Elle est équipée d'un groupe de marqueurs réfléchissants 21, analogues aux marqueurs 5 ou 6, de sorte que l'ensemble ordinateur 2 - capteur 3 permet de visualiser sur l'écran d'affichage 7 la position de la râpe par rapport au fémur. Le chirurgien s'aide ainsi de cette information pour guider la râpe suivant l'axe X-X et creuser la cavité fémorale souhaitée.

A la fin de l'étape de râpage, l'axe X-X d'implantation de la partie fémorale 11 est remplacé par l'axe de râpage effectivement réalisé si celui-ci s'est écarté de l'axe diaphysaire prévu par la broche.

Dans un quatrième temps, indépendant des deuxième et troisième temps décrits ci-dessus et qui peut donc être interverti avec ces derniers, une cavité C globalement hémisphérique est creusée dans la zone du bassin B où est prévue l'implantation du cotyle 16 de la prothèse 10, comme représenté sur la figure 4. Pour permettre à l'ordinateur 2 de connaître les caractéristiques géométriques de la cavité creusée C, plusieurs solutions sont envisageables. Une première solution consiste à équiper la fraise de creusage de la cavité d'un ensemble de marqueurs réfléchissants analogues aux marqueurs 5 ou 6, de manière à enregistrer la progression de cette fraise dans l'os du bassin et ainsi permettre à l'ordinateur 2, qui connaît à l'avance les caractéristiques géométriques de la fraise utilisée, de déterminer notamment la position du centre O de la cavité fraisée. Une autre solution, qui peut être éventuellement combinée à la première, consiste à venir palper la cavité une fois qu'elle est creusée. Une troisième solution consiste à utiliser une cupule fantôme équipée de marqueurs réfléchissants analogues aux marqueurs 5 ou 6 et à venir positionner cette cupule fantôme au fond de la cavité fraisée.

Dans tous les cas, à la fin de cette étape, l'ordinateur connaît la position dans l'espace du centre O de la cavité C, ainsi qu'éventuellement d'autres caractéristiques géométriques relatives à cette cavité, notamment son rayon.

Dans un cinquième temps, on mesure et on mémorise plusieurs configurations d'articulation de la hanche réduite du patient. A cet effet est utilisé un composant fémoral fantôme 22, représenté sur la figure 5, se présentant sous une forme globalement analogue à la partie d'extrémité supérieure de la partie fémorale 11 de la prothèse 10, avec cependant des dimensions plus grandes. Plus précisément, ce composant fémoral 22, appelé par la suite « méga-tête », comporte une tête proprement dite 23 sensiblement hémisphérique d'axe de symétrie Y-Y. La tête 23 est solidaire d'un col 24 essentiellement cylindrique d'axe Y-Y. L'extrémité libre du col 24 est pourvue de moyens de solidarisation à l'extrémité libre supérieure de la râpe 20, laissée en place dans la diaphyse du fémur F à l'issue de l'étape de râpage.

La tête 23 de la méga-tête 22 délimite une surface articulaire 25 sensiblement identique à la surface externe du cotyle 16 de la prothèse 10 à implanter. La tête 23 est ainsi à même d'être articulée directement dans la cavité fraisée C de l'os du bassin B. La position dans l'espace de la méga-tête 22, notamment de son axe Y-Y, est connue de l'ordinateur 2 par l'intermédiaire des capteurs 5 puisque la méga-tête est porté par le manche de la râpe 20 dont la position par rapport au fémur F a été déterminée et mémorisée par l'ordinateur lors du troisième temps de l'intervention.

Alors que la méga-tête 22 est articulée à l'intérieur de la cavité C, le chirurgien sollicite la hanche du patient de manière à ce qu'elle occupe successivement plusieurs configurations considérées comme extrêmes, c'est-à-dire des configurations que la morphologie locale du patient lui impose comme limites naturelles. La hanche du patient est ainsi sollicitée dans une ou plusieurs configurations combinant des mouvements de flexion-extension, abduction-adduction et/ou rotations interneexterne, par exemple dans la configuration en tailleur. Chacune de ces configurations extrêmes caractérise une amplitude articulaire propre à la hanche du patient opéré que la prothèse 10 à implanter est censée par la suite pouvoir reproduire sans courir le risque d'être luxée.

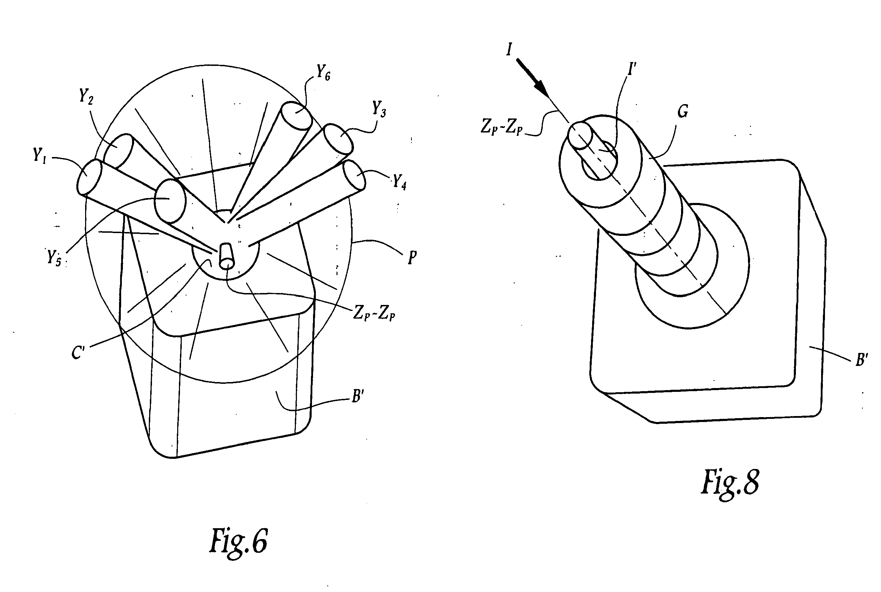

Lorsque le chirurgien sollicite l'articulation de la hanche dans une de ces configurations extrêmes, il actionne la pédale de commande 8 et l'ensemble ordinateur 2 - capteur 3 mesure physiquement et mémorise la position de l'axe Y-Y de la méga-tête 22 par rapport à l'os du bassin B. Comme représenté sur la figure 6, les différentes positions ainsi mesurées réellement, par exemple au nombre de six, sont affichées sur l'écran 7, notamment sous la forme de barres symboliques Y₁, Y₂ ..., Y₆, le bassin B étant schématiquement représenté par un parallélépipède B' et la cavité d'implantation C étant représentée par un creux sensiblement hémisphérique C' correspondant, les représentations graphiques de ces éléments B' et C' étant fonction des mesures antérieures du bassin B et de la cavité C.

Une fois ces mesures effectuées, l'articulation de la hanche est luxée et la méga-tête 22 est retirée.

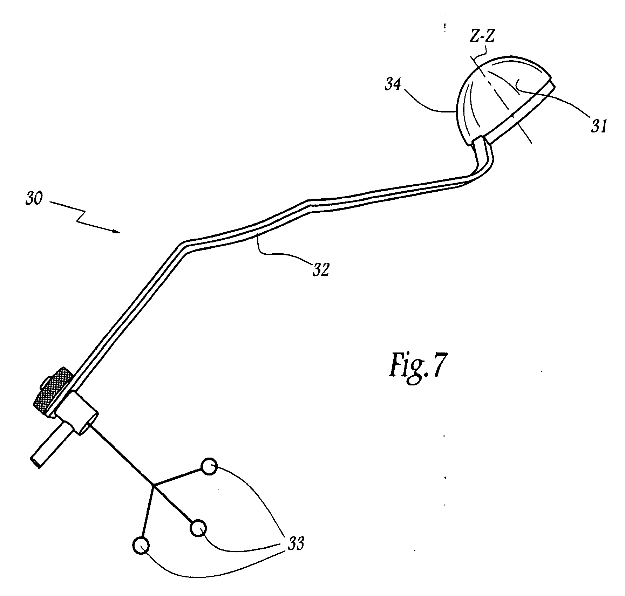

Dans un sixième temps, le chirurgien utilise un ancillaire cotyloïdien 30 représenté seul sur la figure 7, qui comporte un cotyle fantôme 31 relié fixement à un manche rigide de manipulation 32 équipé de marqueurs réfléchissants 33 analogues aux marqueurs 5 ou 6. Le cotyle fantôme 31 se présente sous la forme d'une hémisphère délimitant une surface articulaire convexe 34 sensiblement identique à la surface externe du cotyle 16 de la prothèse 10 à implanter. L'axe de génération de ce cotyle fantôme est noté Z-Z et est repéré en permanence dans l'espace par l'ensemble ordinateur 2 - capteur 3, l'ordinateur connaissant par avance la relation géométrique fixe entre cet axe Z-Z et les marqueurs 33.

Au moyen du manche 32, le cotyle fantôme 31 est manipulé de façon à être logé dans la cavité fraisée C de telle sorte que son axe Z-Z passe sensiblement par le centre O de cette cavité. Comme représenté sur la figure 6, l'ordinateur 2 affiche alors sur son écran 7, en superposition des barres Y₁ à Y₆, le cône de mobilité prothétique P associé à la prothèse 10 à implanter, en fonction de la position effectivement occupée par le cotyle fantôme 31 dans la cavité C, c'est-à-dire en fonction de la position de son axe Z-Z. L'ordinateur 2 connaît en effet par avance les caractéristiques structurelles de la prothèse 10, en particulier l'angle au sommet de son cône de mobilité prothétique, seule la position de l'axe de révolution de ce cône, simulé par l'axe Z-Z du cotyle fantôme 31, étant ajustable par le chirurgien.

Le chirurgien compare alors visuellement la position des barres Y₁ à Y₆ représentatives de la mobilité articulaire maximale de la hanche du patient avec le cône de mobilité prothétique P envisagé selon la position exacte du cotyle fantôme 31 dans la cavité fraisée C. Si, comme sur la figure 6, l'ensemble des barres Y₁ à Y₆ apparaît, au niveau de l'écran d'affichage 7, à l'intérieur du cône P, la position de l'axe Z-Z est considérée comme acceptable, c'est-à-dire que la prothèse 10 ainsi implantée permettra au patient, du point de vue de la mobilité prothétique, de limiter autant que possible les risques de luxations de la prothèse. En revanche si une ou plusieurs des barres Y₁ à Y₆ sont situées à l'extérieur du cône prothétique P, le chirurgien déplace le cotyle fantôme 31 jusqu'à trouver une position dans laquelle les risques de luxations ultérieures de la prothèse 10 sont fortement limités. A cet effet, des informations complémentaires sur les angles respectifs des barres Y₁ à Y₆ et de l'axe Z-Z peuvent être fournies au chirurgien pour lui permettre de trouver rapidement et facilement cette position. De plus, d'autres angles de vue des éléments de la figure 6 sont avantageusement proposés, notamment l'angle sous lequel le cône P apparaît globalement sous forme d'un cercle, la représentation graphique de l'axe Z-Z étant alors dirigée perpendiculairement au plan de vue.

Lorsque le chirurgien a trouvé une position satisfaisante pour le cotyle fantôme 31, il enregistre la position de son axe Z-Z au moyen de l'ordinateur 2, cette direction, notée Zp-Zp sur la figure 6, étant alors choisie comme la direction préférentielle pour implanter ultérieurement la partie cotyloïdienne 12 de la prothèse 10.

En option, en parallèle de ou après la détermination de la direction préférentielle de l'axe Z-Z, il est possible de contrôler cette direction en équipant la râpe 20 d'une tête fémorale d'essai non représentée, de dimensions géométriques sensiblement identiques à la tête fémorale 15 de la prothèse 10. Cette tête d'essai est alors à même d'être articulée à l'intérieur du cotyle fantôme 31 qui reproduit les caractéristiques géométriques internes de l'insert 17 de la prothèse 10. L'articulation de hanche ainsi formée est alors réduite puis sollicitée par le chirurgien selon différentes configurations articulaires extrêmes, afin de vérifier notamment que le col de la tête d'essai n'entre pas en contact avec la matière osseuse du bassin B en provoquant la luxation de la prothèse.

Après avoir retiré le cotyle fantôme 31, le chirurgien utilise ensuite dans un septième temps un impacteur non représenté pour mettre en place de manière définitive le cotyle 16 de la prothèse 10. Pour permettre d'impacter ce cotyle de sorte que son axe C-C coïncide avec la direction préférentielle Zp-Zp, cet ancillaire est équipé de moyens de repérage dans l'espace, permettant à l'ensemble ordinateur 2 - capteur 3 d'afficher sur l'écran 7 la position de sa direction d'impaction I, comme représenté sur la figure 8 sur lequel l'impacteur est symbolisé par un tube I'. Avant d'appliquer l'effort d'impaction au cotyle 16, le chirurgien positionne l'impacteur de manière à ce que la direction I, dans le prolongement de l'axe C-C du cotyle, soit sensiblement alignée avec la position préférentielle Zₚ-Zₚ de l'axe du cône de mobilité prothétique. A cet effet, l'ordinateur 2 affiche un tube virtuel de guidage G, partiellement évidé, à l'intérieur duquel la représentation symbolique I' de l'impacteur doit être placée de façon coaxiale pour assurer l'alignement des directions I et Zₚ-Zₚ. Un signalement visuel, tel qu'un changement de couleur ou un clignotement, signale l'alignement au chirurgien.

L'insert 17 est ensuite logé dans le cotyle implanté.

Une fois l'impaction réalisée, tous les composants fémoraux du dispositif 1 sont retirés et la partie fémorale 11 de la prothèse 10 est, dans un huitième temps, implantée de manière à ce que l'axe A-A de sa tige 13 soit disposé de manière sensiblement confondue avec l'axe d'implantation fémorale X-X. Dans la mesure où la râpe 20 a ménagé une cavité diaphysaire sensiblement complémentaire de cette tige, il est suffisant d'impacter de manière classique la tige 13 dans le fémur F pour obtenir la coïncidence des axes A-A et X-X.

Le dispositif 1 selon l'invention permet ainsi de positionner la prothèse 10 de façon optimale pour reproduire autant que possible les capacités cinématiques de la hanche anatomique du patient opéré. On notera que les huit temps peropératoires décrits ci-dessus sont effectués au cours d'une intervention chirurgicale proprement dite, c'est-à-dire durant laquelle le patient est par exemple sous anesthésie.

De plus, les différentes données enregistrées durant la pose de la prothèse 10 peuvent être utilisées pour dresser un bilan post-opératoire et permettre ainsi de caractériser avec précision les capacités articulaires de la prothèse dans son état d'implantation dans les os de la hanche. Il est également possible de déterminer l'allongement entre le fémur F et le bassin B durant l'intervention chirurgicale. On remarquera cependant que les données acquises sont nettement moins nombreuses que celles nécessaires pour le fonctionnement d'un simulateur biomécanique de la hanche à opérer et les moyens informatiques correspondants du dispositif selon l'invention sont donc moins onéreux et moins complexes à manipuler.

Comme indiqué plus haut, le dispositif d'implantation 1 est en outre facilement applicable à des prothèses de différentes géométries, seules les caractéristiques de mobilité prothétique étant à fournir à l'ordinateur 2 pour permettre d'afficher le cône P. Des jeux correspondants de fraises et de râpes sont prévus, ainsi qu'un jeu de plusieurs cotyles fantômes 31 de tailles et de géométries différentes, à même d'être solidarisés à un même manche 32.

Divers aménagements et variantes au dispositif d'implantation 1 décrit ci-dessus sont en outre envisageables.

En particulier, l'utilisation de l'ancillaire cotyloïdien 30 n'est pas indispensable puisque les données relatives au cône de mobilité prothétique P sont connues à l'avance de l'ordinateur 2, seule la position de l'axe Z-Z par rapport à la cavité C du bassin B étant à ajuster en cours d'intervention pour garantir le fonctionnement ultérieur sans luxation de la prothèse 10. Il est donc envisageable que le chirurgien n'utilise que des représentations virtuelles pour ajuster la position de cet axe Z-Z, en affichant les différents cônes de mobilité prothétique qui correspondent à différentes positions de l'axe Z-Z, par exemple au moyen d'une interface informatique appropriée lui permettant de modifier la position de l'axe virtuel Z-Z et de choisir l'axe préférentiel Zₚ-Zₚ.

D'autres variantes sont énumérées ci-dessous :
- pour supporter la méga-tête 22, le manche de râpe 20 peut être remplacé par une broche fémorale ou par la tige fémorale de la prothèse à implanter, la méga-tête pouvant alors éventuellement être venue de matière avec son support fémoral ;
- la méga-tête 22 décrite ci-dessus peut être remplacée par un composant fémoral fantôme constitué d'une tête de dimensions sensiblement identiques à celles de la tête fémorale prothétique et d'une calotte hémisphérique articulée sur cette tête et de dimensions sensiblement identiques à celles de la partie cotyloïdienne prothétique ;
- les moyens de repérage des os du fémur F et du bassin B ne sont pas limités à des marqueurs réfléchissant l'infra-rouge, des marqueurs sensibles aux ultra-sons ou aux champs électromagnétiques étant par exemple utilisables ;
- la cavité C peut être fraisé après avoir déterminé la direction préférentielle Zp-Zp ; on utilise dans ce cas la cavité anatomique de la hanche comme logement articulaire de la méga-tête 22 pour mesurer les différentes configurations articulaires extrêmes ;
- d'autres moyens qu'un écran d'affichage sont envisageables pour communiquer au chirurgien un retour d'informations sur la comparaison entre les mesures des configurations articulaires extrêmes et le cône de mobilité prothétique ; des indications sonores ou tactiles peuvent ainsi faire comprendre au chirurgien l'état de cette comparaison et le guider dans la détermination de la direction préférentielle Zp-Zp ; et/ou
- la détermination de la direction préférentielle Zp-Zp peut être intégralement assurée par un logiciel approprié équipant l'ordinateur 2, à partir de la comparaison du cône P et des mesures des différentes configurations articulaires extrêmes mesurées en peropératoire, et ce par calcul et extrapolation.

## Revendications

1. Dispositif chirurgical d'implantation d'une prothèse totale de hanche (10) ayant un cône de mobilité (P) avec un axe de révolution (Z-Z), ce dispositif comportant des moyens de mesure (2, 3, 22) pour mesurer de manière peropératoire plusieurs positions d'une direction prothétique fémorale donnée (Y-Y) et des moyens de fourniture (2, 3, 30) pour fournir de manière peropératoire la position dans l'espace de l'axe de révolution (Z-Z) du cône de mobilité (P), **caractérisé par** des moyens (2) de mémorisation desdites positions de la direction prothétique fémorale (Y-Y) et par des moyens (2) de comparaison peropératoire de ces positions avec le cône (P) de mobilité de la prothèse (10) à implanter, afin de pouvoir ajuster dans l'espace la position de l'axe de révolution (Z-Z) de ce cône lors de l'implantation de la prothèse, de telle sorte que lesdites positions de la direction prothétique fémorale (Y-Y) soient situées à l'intérieur du cône (P).

2. Dispositif suivant la revendication 1, **caractérisé en ce qu'**il comporte des moyens (7) de communication au chirurgien de la comparaison entre les positions de la direction prothétique fémorale (Y-Y) et le cône de mobilité prothétique (P).

3. Dispositif suivant la revendication 2, **caractérisé en ce que** les moyens de communication comportent un moyen d'affichage (7) de représentations symboliques (Y₁, ... Y₆, P) des positions de la direction prothétique fémorale (Y-Y) et de la position du cône de mobilité prothétique.

4. Dispositif suivant l'une quelconque des revendications précédentes, **caractérisé en ce qu'**il comporte des moyens de sélection (2, 8) par le chirurgien ou de calcul et de mémorisation d'au moins une position préférentielle (Zₚ-Zₚ) de l'axe (Z-Z) du cône de mobilité prothétique (P).

5. Dispositif suivant la revendication 4, **caractérisé en ce qu'**il comporte en outre, d'une part, un impacteur de mise en place définitive, dans la zone d'implantation (C) du bassin (B), du cotyle (16) de la prothèse (10), équipé de moyens de repérage dans l'espace et, d'autre part, des moyens (2) de comparaison de la direction d'impaction (I) de cet impacteur avec la position préférentielle (Zₚ-Zₚ).

6. Dispositif suivant l'une quelconque des revendications précédentes, **caractérisé en ce que** les moyens de mesure comportent un composant fémoral fantôme (22) définissant la direction prothétique fémorale (Y-Y), de sorte que les positions de la direction prothétique fémorale (Y-Y) à comparer au cône de mobilité prothétique (P) correspondent à des positions de ce composant fémoral dans l'espace, notamment par rapport à une zone (C) du bassin (B) où est prévue l'implantation d'un cotyle (16) de la prothèse.

7. Dispositif suivant la revendication 6, **caractérisé en ce qu'**il comporte des moyens de détermination du centre (O) d'une cavité osseuse (C) du bassin (B), constituant la zone d'implantation du bassin et apte à recevoir le composant fémoral fantôme (22) lors de la mesure des différentes positions de ce composant.

8. Dispositif suivant l'une des revendications 6 ou 7, **caractérisé en ce qu'**il comporte un support (20) pour le composant fémoral fantôme (22), adapté pour être relié fixement au fémur (F) et pour porter des moyens (21) de repérage dans l'espace.

9. Dispositif suivant l'une quelconque des revendications 6 à 8, **caractérisé en ce que** le composant fémoral fantôme (22) délimite une surface articulaire (25) apte à être articulée directement sur la zone d'implantation (C) du bassin (B) et sensiblement identique à la surface externe du cotyle (16) de la prothèse (10) à implanter.

10. Dispositif suivant l'une quelconque des revendications précédentes, **caractérisé en ce que** les moyens de fourniture comportent un cotyle fantôme (31) reproduisant l'axe (Z-Z) du cône de mobilité prothétique (P) et équipé d'un moyen (32) de manipulation manuelle dans l'espace, notamment par rapport à la zone d'implantation (C) du bassin (B), pourvu de moyens (33) de repérage dans l'espace.

## Claims

1. Surgical device for implanting a total hip prosthesis (10) having a cone of mobility (P) with an axis of revolution (Z-Z), this device comprising measuring means (2, 3, 22) for per-operatively measuring a plurality of positions of a given femoral prosthetic direction (Y-Y) and providing means (2, 3, 30) for per-operatively providing the position in space of the axis of revolution (Z-Z) of the cone of mobility (P), **characterised by** means (2) for memorizing the said positions of the femoral prosthetic direction (Y-Y), and by means (2) for per-operatively comparing of the said positions with the cone of mobility (P) of the prosthesis (10) to be implanted, so as to adjust in space the position of the axis of revolution (Z-Z) of this cone during the implantation of the prosthesis, so that said positions of the femoral prosthetic direction (Y-Y) are located within the cone (P).

2. The device of Claim 1, **characterized in that** it comprises means (7) for communicating to the surgeon the comparison between the positions of the femoral prosthetic direction (Y-Y) and the cone (P) of mobility of the prosthesis.

3. The device of Claim 2, **characterized in that** the communication means comprise a means for displaying (7) symbolic representations (Y₁, ... Y₆, P) of the positions of the femoral prosthetic direction (Y-Y) and of the position of the cone of mobility of the prosthesis.

4. The device of any one of the preceding Claims, **characterized in that** it comprises means (2, 8) for selection by the surgeon or for calculation and memorization of at least one preferential position (Zₚ-Zₚ) of the axis (Z-Z) of the cone (P) of mobility of the prosthesis.

5. The device of Claim 4, **characterized in that** it further comprises, on the one hand, an impactor for definitively positioning the acetabulum (16) of the prosthesis (10) in the zone of implantation (C) of the pelvis (B), equipped with means for locating in space and, on the other hand, means (2) for comparing the direction of impaction (I) of this impactor with the preferential position (Zₚ-Zₚ).

6. The device of any one of the preceding Claims, **characterized in that** the measuring means comprise a phantom femoral component (22) defining the femoral prosthetic direction (Y-Y), so that the said positions of the femoral prosthetic direction (Y-Y) to be compared with the prosthetic cone of mobility (P) correspond to positions of this femoral component in space, especially with respect to a zone (C) of the pelvis (B) where the implantation of an acetabulum (16) of the prosthesis is provided.

7. The device of Claim 6, **characterized in that** it comprises means for determining the centre (O) of an osseous cavity (C) of the pelvis (B), constituting the zone of implantation of the pelvis and adapted to receive the phantom femoral component (22) when the different positions of this component are measured.

8. The device of one of Claims 6 or 7, **characterized in that** it comprises a support (20) for the phantom femoral component (22), adapted to be fixedly connected to the femur (F) and to bear means (21) for locating in space.

9. The device of any one of Claims 6 to 8, **characterized in that** the phantom femoral component (22) defines an articular surface (25) adapted to be directly articulated on the zone of implantation (C) of the pelvis (B) and substantially identical to the outer surface of the acetabulum (16) of the prosthesis (10) to be implanted.

10. The device of any one of the preceding Claims, **characterized in that** the providing means comprise a phantom acetabulum (31) reproducing the axis (Z-Z) of the cone (P) of mobility of the prosthesis and equipped with a means (32) for manual manipulation in space, especially with respect to the zone of implantation (C) of the pelvis (B), provided with means (33) for locating in space.

## Patentansprüche

1. Chirurgische Vorrichtung für die Implantation einer Hüfttotalvollprothese (10), die einen Beweglichkeitskonus (P) mit einer Drehachse (Z-Z) besitzt, wobei diese Vorrichtung Messmittel (2, 3, 22) aufweist, um präoperativ mehrere Positionen einer gegebenen Oberschenkelprothesenrichtung (Y-Y) zu messen, und Bereitstellungsmittel (2, 3, 30) aufweist, um präoperativ die Position im Raum der Symmetrieachse (Z-Z) des Beweglichkeitskonus (P) zu liefern, **gekennzeichnet durch** Mittel (2) zum Speichern der genannten Positionen der Oberschenkelprothesenrichtung (Y-Y) und Mittel (2) zum präoperativen Vergleichen dieser Positionen mit dem Beweglichkeitskonus (P) der zu implantierenden Prothese (10), um im Raum die Position der Drehachse (Z-Z) dieses Konus bei der Implantation der Prothese einstellen zu können, derart, dass sich die genannten Positionen der Oberschenkelprothesenrichtung (Y-Y) innerhalb des Konus (P) befinden.

2. Vorrichtung nach Anspruch 1, **dadurch gekennzeichnet, dass** sie Mittel (7) für die Übermittlung des Vergleichs zwischen den Positionen der Oberschenkelprothesenrichtung (Y-Y) und dem Prothesenbeweglichkeitskonus (P) an den Chirurgen enthält.

3. Vorrichtung nach Anspruch 2, **dadurch gekennzeichnet, dass** die Übermittlungsmittel ein Mittel (7) zum Anzeigen symbolischer Darstellungen (Y₁, ..., Y₆, P) der Positionen der Oberschenkelprothesenrichtung (Y-Y) und der Position des Prothesenbeweglichkeitskonus enthalten.

4. Vorrichtung nach einem der vorhergehenden Ansprüche, **dadurch gekennzeichnet, dass** sie Mittel (2, 8) für die Auswahl durch den Chirurgen oder für die Berechnung und die Speicherung wenigstens einer bevorzugten Position (Zₚ-Zₚ) der Achse (Z-Z) des Prothesenbeweglichkeitskonus (P) enthält.

5. Vorrichtung nach Anspruch 4, **dadurch gekennzeichnet, dass** sie außerdem einerseits eine Schlageinrichtung für die endgültige Anordnung der Gelenkpfanne (16) der Prothese (10) in der Implantationszone (C) des Beckens (B), die mit Mitteln zum Orientieren im Raum ausgerüstet ist, und andererseits Mittel (2) zum Vergleichen der Schlagrichtung (I) dieser Schlageinrichtung mit der bevorzugten Position (Zₚ-Zₚ) enthält.

6. Vorrichtung nach einem der vorhergehenden Ansprüche, **dadurch gekennzeichnet, dass** die Messmittel eine Phantomoberschenkelkomponente (22) enthalten, die die Oberschenkelprothesenrichtung (Y-Y) definiert, derart, dass die Positionen der Oberschenkelprothesenrichtung (Y-Y), die mit dem Prothesenbeweglichkeitskonus (P) zu vergleichen ist, Positionen dieser Oberschenkelkomponente im Raum entsprechen, insbesondere in Bezug auf eine Zone (C) des Beckens (B), wo die Implantation einer Gelenkpfanne (16) der Prothese vorgesehen ist.

7. Vorrichtung nach Anspruch 6, **dadurch gekennzeichnet, dass** sie Mittel zum Bestimmen des Zentrums (O) eines Knochenhohlraums (C) des Beckens (B) enthält, das die Implantationszone des Beckens bildet und die Phantomoberschenkelkomponente (22) aufnehmen kann, wenn verschiedene Positionen dieser Komponente gemessen werden.

8. Vorrichtung nach einem der Ansprüche 6 oder 7,
**dadurch gekennzeichnet, dass** sie einen Träger (20) für die Phantomoberschenkelkomponente (22) enthält, der dazu ausgelegt ist, mit dem Oberschenkel (F) fest verbunden zu werden und Mittel (21) für die Orientierung im Raum zu tragen.

9. Vorrichtung nach einem der Ansprüche 6 bis 8, **dadurch gekennzeichnet, dass** die Phantomoberschenkelkomponente (22) eine Gelenkoberfläche (25) begrenzt, die dazu ausgelegt ist, direkt an der Implantationszone (C) des Beckens (B) angelenkt zu werden und im Wesentlichen mit der äußeren Oberfläche der Gelenkpfanne (16) der zu implantierenden Prothese (10) übereinstimmt.

10. Vorrichtung nach einem der vorhergehenden Ansprüche, **dadurch gekennzeichnet, dass** die Liefermittel eine Phantomgelenkpfanne (31) enthalten, die die Achse (Z-Z) des Prothesenbeweglichkeitskonus (P) wiedergibt und mit einem Mittel (32) für die manuelle Manipulation im Raum, insbesondere in Bezug auf die Implantationszone (C) des Beckens (B) ausgerüstet ist, das mit Mitteln (33) für die Orientierung im Raum versehen ist.
